# FASCICULE DE BREVET EUROPEEN

(11) **EP 0 511 964 B1**
(45) Date de publication et mention de la délivrance du brevet: **15.10.1997**
(21) Numéro de dépôt: 91900265.9
(22) Date de dépôt: 23.11.1990
(51) Int. Cl.: C07K 1/00, C12N 15/26, C12P 21/02

(54) **PROCEDE DE PREPARATION D'UNE PROTEINE CHOISIE PARMI LES CYTOKINES, COMPORTANT AU MOINS UN PONT DISULFURE INTRAMOLECULAIRE PAR OXYDATION, A UN pH INFERIEUR A 5,0, DE LA PROTEINE RECOMBINANTE REDUITE CORRESPONDANTE**
VERFAHREN ZUR HERSTELLUNG EINES PROTEINS GEHÖREND ZU DER KLASSE DER CYTOKINE, DAS MINDESTENS EINE INTRAMOLEKULARE DISULFIDBRÜCKE ENTHÄLT, DURCH OXIDATION BEI EINEM PH-WERT KLEINER ALS 5.0 DES ENTSPRECHENDEN REKOMBINANTEN REDUZIERTEN PROTEINS
METHOD FOR PREPARING A PROTEIN SELECTED FROM THE CYTOKINE GROUP, COMPRISING AT LEAST ONE INTRAMOLECULAR DISULPHIDE BOND BY OXIDATION, AT A pH OF LESS THAN 5.0, OF THE REDUCED RECOMBINANT PROTEIN

(43) Date de publication de la demande: 11.11.1992
(73) Titulaire: ROUSSEL UCLAF, 93230 Romainville (FR)
(72) Inventeur: ABECASSIS, Pierre-Yves, F-75020 Paris (FR); LECLAIRE, Jacques, F-91300 Massy (FR); RIBERON, Philippe, F-94130 Nogent-sur-Marne (FR)
(74) Mandataire: Vieillefosse, Jean-Claude
(86) Numéro de dépôt international: FR9000843
(87) Numéro de publication internationale: WO9209623

(56) Documents cités:
- EP-A- 0 185 459
- EP-A- 0 360 937
- Methods in Enzymology, vol. 107, 1984, Academic Press, Inc., (New York, US) T.E. Creighton: "Disulfide bond formation in proteins", p. 305-329

## Description

La présente invention concerne un procédé de préparation d'une protéine choisie parmi les cytokines, comportant au moins un pont disulfure intramoléculaire par oxydation, à un pH inférieur à 5,0, de la protéine recombinante réduite correspondante.

De nombreuses protéines recombinantes à usage thérapeutique sont exprimées dans des systèmes hôtes variés parmi lesquels E. Coli, la levure et les cellules CHO représentent ceux les plus fréquemment utilisés.

Parmi ces protéines, les protéines hétérologues exprimées mais non sécrétées par exemple dans la levure ou dans E. Coli sont en général accumulées sous forme d'aggrégats dénaturés à l'état de granules insolubles. Les procédés d'extraction nécessitent une mise en solution en milieu dénaturant à l'aide d'agents tels que l'urée ou la guanidine. Le repliement correct de la molécule, qui peut être ensuite réalisé par des méthodes variées connues et qui est nécessaire pour que la protéine manifeste son activité biologique, n'est pas toujours obtenu de façon sélective et conduit à des rendements variables, souvent faibles, selon la protéine recombinante concernée en raison de la formation associée de conformères non actifs qu'il faut séparer par des techniques de purification connues, par exemple par chromatographie.

En particulier lorsque la protéine renferme au moins un pont disulfure intramoléculaire, et éventuellement une ou plusieurs cystéines supplémentaires, la protéine recombinante non sécrétée est généralement produite à l'état réduit dans les granules. Ainsi lorsque le rétablissement d'un ou plusieurs ponts disulfure spécifiques, soit nécessaire à l'activité biologique de la protéine, soit qui contribue à une augmentation de cette activité est désiré, le procédé d'oxydation doit assurer le repliement correct de la protéine sans formation des éventuels isomères indésirables correspondants à la formation au hasard de ponts intramoléculaires ou intermoléculaires.

Une mise au point concernant les réactions régiosélectives permettant la formation de ponts disulfure à partir de cystéines dans des chaînes peptidiques, obtenues par synthèse chimique, décrit une multiplicité de méthodes qui met en évidence la difficulté à optimiser les rendements d'une façon générale et l'absence de résultats quantitatifs (F, Cavelier et al. Bull. Soc. Chim. Franc. 1989 n°6 788-798).

Des procédés d'oxydation contrôlée pour les protéines produites par la technique du génie génétique ont été décrits, notamment dans le domaine des cytokines recombinantes renfermant au moins 2 cystéines et produites dans E. Coli, par exemple pour l'interféron β et l'interleukine 2 (IL2) dont les protéines naturelles correspondantes renferment 3 cystéines et un pont disulfure ayant une position spécifique :
. le brevet US 4,530,787 correspondant à la demande européenne EP0156354 décrit un procédé d'oxydation de protéines recombinantes complètement réduites illustré par l'application à une mutéine réduite de l'interféron β qui renferme 2 cystéines (exemple 1), à la desAla-IL2 réduite qui renferme 3 cystéines (exemple 3) ou à une mutéine réduite de la desAla-IL2 qui renferme 2 cystéines (exemple 4), à l'aide d'iodosobenzoate à un pH compris entre 5,5 et 9,0, avec lequel on observe selon les conditions opératoires jusqu'à 10 à 15 % d'oligomères oxydés non désirés formés.
. le brevet US 4,572,798 correspondant à la demande européenne EP0185459 décrit une oxydation contrôlée à l'aide de chlorure de cuivre à un pH compris entre 5,5 et 9 et notamment à pH 8,0 en présence d'air à 25°C (exemple 2) ou à 37°C (exemple 3) de la mutéine de la desAla-IL2 ci-dessus dont l'application à la desAla-IL2 réduite ci-dessus conduit à la formation d'environ 15 % de produits oxydés inactifs ayant des ponts disulfure isomères non désirés.
. M, P, Weir et al. (Biochem. J 1987 245 85-91) décrit un procédé d'oxydation de l'IL2 recombinante exprimée dans E. Coli en solution aqueuse à la concentration de 1 à 2 µg/ml, en présence de guanidine, par action de sulfate cuivrique à pH égal à 8,5 qui nécessite ensuite une purification par chromatographie en phase inversée pour obtenir l'IL2 purifiée. De plus les auteurs indiquent que l'oxydation devient "extrêmement lente" lorsque le pH est inférieur à 6.

Ainsi en l'absence de méthode d'oxydation sélective quantitative des protéines recombinantes réduites comprenant au moins 2 cystéines, il est nécessaire après oxydation à un pH supérieur à 5,0 de faire une purification, en général par chromatographie, qui élimine les produits d'oxydation correspondants à la formation incorrecte de ponts intramoléculaires isomères ainsi que de ponts intermoléculaires.

L'obtention d'une protéine recombinante oxydée homogène pose donc des problèmes techniques de purification qui conduit à un abaissement du rendement en produit recherché.

Or il vient d'être découvert que, de façon surprenante, le procédé de préparation d'une protéine recombinante, comprenant au moins un pont disulfure, ne nécessite pas de stade de purification ultérieur pour obtenir la protéine oxydée désirée homogène et permet donc d'obtenir la protéine avec un rendement amélioré si l'oxydation est conduite à pH acide, inférieur à 5,0.

L'objet de la présente invention concerne donc un procédé de préparation d'une protéine choisie parmi les cytokines, comportant au moins un pont disulfure intramoléculaire, et éventuellement une ou plusieurs cystéines supplémentaires, comprenant l'oxydation de la protéine recombinante réduite correspondante à l'aide d'un agent d'oxydation et caractérisé en ce que l'on fait réagir une solution aqueuse de la protéine réduite à un pH inférieur à 5,0.

La protéine concernée par l'invention est une protéine choisie parmi les polypeptides ayant une séquence en acides aminés naturelle ou synthétique, renfermant au moins un pont disulfure intramoléculaire qui contribue à l'activité biologique de la protéine et éventuellement une ou plusieurs cystéines libres supplémentaires non engagées dans des ponts disulfure, par exemple, des cytokines, que l'on produit par la technologie de l'ADN recombinant et isole à l'état réduit, selon les méthodes générales connues, avant le stade d'oxydation à pH acide qui fait l'objet de l'invention.

Par protéine naturelle on entend une protéine qui peut être isolée à partir de sources naturelles. Il est évident, en effet, que le procédé est d'application tout à fait générale et peut être mis en oeuvre pour la préparation de toute protéine choisie parmi les cytokines répondant aux critères ci-dessus.

Par état réduit on entend que les cystéines que contient la protéine renferment un groupement sulfhydryle libre dont la détermination peut être faite par exemple par spectrophotométrie avec la dithiodipyridine comme réactif des thiols.

L'agent d'oxydation utilisé pour la mise en oeuvre de l'invention est choisi parmi les agents d'oxydation connus qui génèrent un atome d'oxygène activé,
soit directement tel qu'un dérivé d'iodosylaryle, par exemple un acide iodosobenzoïque,
soit indirectement tel que l'iode en présence d'eau ou tel qu'un complexe cuivrique, par exemple le complexe cuivrique formé avec l'o-phénanthroline, ou un sel cuivrique, par exemple le sulfate cuivrique, en présence d'air,
et à l'action duquel on soumet, éventuellement en présence d'air, la protéine recombinante réduite. On utilise de préférence comme agent d'oxydation l'acide o.iodosobenzoïque sous une atmosphère d'argon ou le sulfate cuivrique en présence d'air.

La concentration de l'agent d'oxydation varie selon l'agent utilisé. Par exemple, lorsque l'agent est le donneur direct d'oxygène tel que l'acide o.iodosobenzoïque, la concentration est dans un rapport au moins stoechiométrique avec la concentration en pont disulfure à former et lorsque l'agent active directement l'oxygène, par exemple l'oxygène de l'air, tel que le sulfate cuivrique, la concentration est inférieure ou égale à la concentration équivalente en protéine à oxyder.

La température du mélange réactionnel peut être choisie par exemple entre 15°C et 40°C, de préférence à environ 37°C.

La durée de la réaction d'oxydation dépend des conditions opératoires ci-dessus. D'une façon générale la réaction est terminée en moins de 3 heures, de préférence environ 1 heure.

A la fin de la réaction, la protéine oxydée désirée homogène obtenue peut être directement récupérée selon des méthodes connues, par exemple par lyophilisation.

Le procédé de l'invention concerne particulièrement un procédé caractérisé en ce que la protéine réduite a la séquence en acides aminés d'une protéine naturelle, d'allèles ou de dérivés de celle-ci.

Par allèles ou dérivés, on inclut les séquences modifiées par substitution ou délétion de un ou plusieurs acides aminés autres que les cystéines qui forment le ou les ponts disulfure que comporte naturellement la protéine désirée aussi bien que l'addition de un ou plusieurs acides aminés, y compris de cystéines qui peuvent éventuellement contribuer à la formation de ponts disulfure qui n'existent pas dans la protéine naturelle pour autant que ces produits conservent l'activité biologique de la protéine naturelle. L'obtention de telles modifications est bien connue dans la méthode de l'ADN recombinant, par exemple par les techniques de la mutagénèse dirigée.

Le procédé de l'invention concerne un procédé caractérisé en ce que la protéine est choisie parmi les cytokines, de préférence l'IL2. Les cytokines de l'invention peuvent être soit des cytokines dont la protéine naturelle comporte un seul pont disulfure, par exemple le TNF alpha ou l'interleukine 3, ou plusieurs ponts disulfure, par exemple l'interféron alpha, l'interleukine 6 ou le GM-CSF, et éventuellement comporte une ou plusieurs cystéines supplémentaires, par exemple l'interféron β, le G-CSF, l'interleukine 4 ou l'IL2,
soit des allèles ou dérivés de celles-ci, par exemple des mutéines de l'IL2 telles que décrites dans le brevet EP 0109748 B ou des dérivés de l'IL2 ayant par exemple une délétion d'un ou plusieurs acides aminés à l'extrémité N-terminale tels que décrits dans la demande de brevet EP 0219839.

Les cytokines réduites nécessaires à la mise en oeuvre de l'invention sont des protéines, soit directement produites et isolées à l'état réduit à partir de la souche hôte et stabilisée en milieu acide par exemple selon le procédé décrit dans la demande de brevet EP 0353150, soit que l'on prépare par réduction totale de la protéine oxydée ou d'un mélange de la protéine oxydée et de la protéine réduite, renfermant éventuellement des ponts isomères indésirés que l'on peut effectuer selon des méthodes connues à l'aide d'un agent réducteur tel que le dithiothreitol ou le mercaptoéthanol.

Le procédé de l'invention est spécialement caractérisé en ce que le pH est compris entre 0,5 et 5,0, de préférence environ 3,0. Le pH désiré de la solution réactionnelle peut être obtenu à l'aide soit d'un acide minéral par exemple l'acide chlorhydrique ou l'acide borique soit d'un acide organique choisi parmi les acides monocarboxyliques ou polycarboxyliques tel que l'acide formique, acétique, propionique, trifluoroacétique, oxalique ou citrique, de préférence l'acide acétique.

Le procédé est également caractérisé en ce que la concentration de la protéine est comprise entre 0,05 mg/ml et 50 mg/ml, de préférence entre 0,1 et 10 mg/ml.

Le procédé de l'invention concerne plus spécialement un procédé caractérisé en ce que la protéine est une IL2 humaine recombinante réduite non glycosylée ayant au moins 2 cystéines en position 58 et 105.

L'IL2 ci-dessus ayant au moins 2 cystéines en position 58 et 105 peut être éventuellement une mutéine recombinante de l'IL2 naturelle dans laquelle la cystéine en position 125 a été substituée par un autre acide aminé, par exemple la sérine telle que l'IL2-Ser125 qui a été décrite (G. Ju et al, J. Biol. Chem. 262 n°12 april 25 5723-5731) et dont la préparation sous forme réduite peut être obtenue par exemple selon le procédé décrit dans la demande de brevet EP 0353150.

Le procédé de l'invention concerne tout spécialement un procédé caractérisé en ce que la protéine est une IL2 humaine recombinante réduite non glycosylée ayant 3 cystéines en position 58, 105 et 125 et qui manifeste une activité biologique comparable à celle de l'IL2 oxydée correspondante ayant un pont disulfure en position 58-105. Une telle IL2 et un exemple de préparation de celle-ci sont décrits dans la demande de brevet EP 0353150.

Les conditions préférées du procédé de l'invention sont caractérisées en ce que l'on fait réagir l'IL2 réduite ci-dessus en solution à environ 1 mg/ml, à un pH environ égal à 3,0, à une température environ égale à 37°C, en présence d'air avec du sulfate cuivrique à une concentration à environ 66 µM. A la fin de la réaction qui peut être suivie par une méthode analytique telle que la mesure de la consommation en oxygène à l'aide d'une électrode de Clark ou une chromatographie liquide haute performance en phase inversée (RP-HPLC) qui sépare la forme oxydée de la forme réduite de départ, la solution obtenue de l'IL2 attendue peut être ensuite conservée par exemple à 4°C ou lyophilisée directement et éventuellement faire l'objet d'une formulation pharmaceutique selon des méthodes connues.

Toutes les publications qui sont citées sont incorporées, par référence, dans le texte de la présente demande.

Les figures ci-annexées illustrent le procédé décrit plus loin :

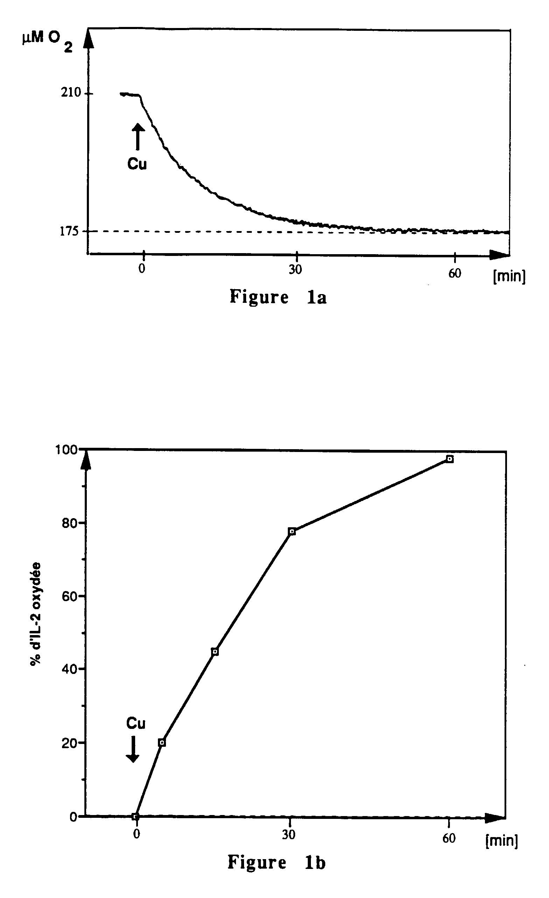

La figure 1a est la courbe de la concentration en µMoles d'oxygène dissous de la solution réactionnelle de l'exemple 1 en fonction du temps et la figure 1b est la courbe de pourcentage d'IL2 oxydée en fonction du temps, calculée d'après la concentration en oxygène dissous.

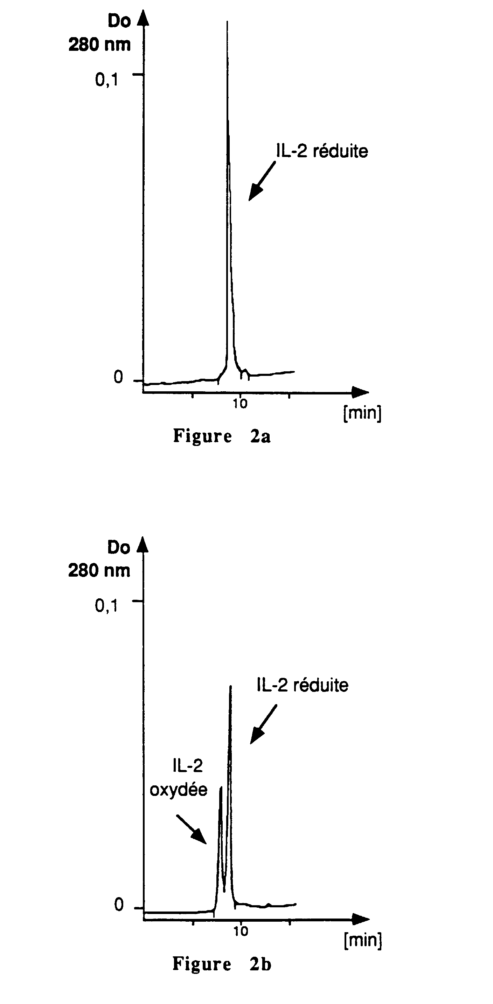

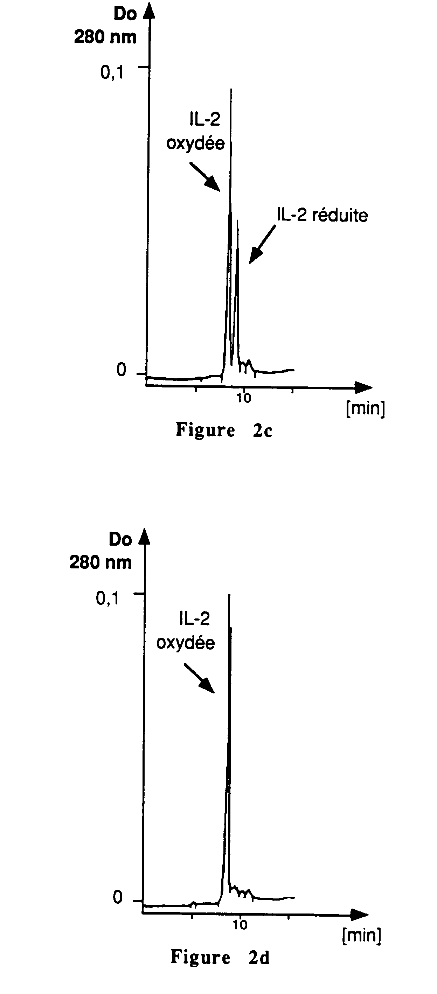

Les figures 2a à 2d représentent 4 chromatogrammes de RP-HPLC analytique de la solution réactionnelle de l'exemple 1 en fonction du temps : la figure 2a représente la solution à t = 0 mn, la figure 2b représente la solution à t = 15 mn, la figure 2c représente la solution à t = 30 mn, la figure 2d représente la solution à t = 60 mn.

L'exemple suivant illustre l'invention sans la limiter :

### EXEMPLE 1 : Oxydation d'IL2 humaine recombinante non glycosylée (r-hIL2) réduite.

### 1) Partie expérimentale :

La r-hIL2 réduite de départ est une fraction "59" préparée selon le procédé décrit à l'exemple 1 ou 2 de la demande de brevet EP 0353150 dans lequel on remplace l'acide citrique du gradient linéaire d'isopropanol (20 à 70 % sur 40 mn) par de l'acide trifluoracétique (TFA) à la concentration de 0,1 % et que l'on a immédiatement lyophilisée à raison d'1 mg d'IL2 réduite par flacon-dose.

L'oxydation de 1 mg de l'IL2 réduite ci-dessus est réalisée à 37°C et à pH # 3 par mise en solution dans 1 ml d'une solution aqueuse d'acide acétique à la concentration de 0,1 % dans laquelle on ajoute 100 µl d'une solution 0,67 mM de sulfate cuivrique. La cinétique de l'oxydation est suivie d'une part par mesure en continu de la consommation en oxygène à l'aide d'une électrode de Clark (figure 1a), d'autre part par RP-HPLC analytique sur une colonne C4 VYDAC (0,46 x 15 cm) 300 A°, 5 microns, au débit de 2 ml/mn, avec un gradient linéaire d'acétonitrile (30 à 70 % sur 10 mn) contenant 0,1 % de TFA et une détection spectrophotométrique à 280 nm dont on évalue la surface des pics respectifs de l'IL2 réduite de départ éluée à environ 60 % d'acétonitrile (figure 2a) et de l'IL2 oxydée éluée à environ 57 % d'acétonitrile formée au cours de la réaction (figures 2b à 2d).

Les figures ci-dessus montrent que l'IL2 est oxydée à environ 40 % à t = 15 mn (figure 2b), à environ 60 % à t = 30 mn (figure 2c) et à environ 100 % à t = 60 mn (figure 2d).

La solution réactionnelle après oxydation à t = 60 mn est ensuite soumise directement aux différentes analyses.

### 2) Résultat des analyses :

- la teneur en groupement sulfhydryle déterminée par spectrophotométrie à 343 nm avec la dithiodipyridine comme réactif des thiols est de 0,85 SH/mole d'IL2 oxydée obtenue, comparée à une teneur de 2,87 SH/mole d'IL2 réduite de départ.
- la localisation de pont disulfure déterminée selon la méthode décrite par Yamada et al. (Arch. Biochem. Biophys. 1987, 257, 194-199) conclue à la présence d'un pont disulfure en position 58-105 et d'un thiol libre en position 125.
- l'activité biologique déterminée par mesure de la prolifération de lignées cellulaires leucémiques de souris dépendantes de l'IL2 CTLL-2, avec un test colorimétrique au sel de tétrazolium (Mossmann, T J. Immunol. Meth. 1983 65 55-63), est de 2,0 x 10⁷ U BRMP/mg, comparée à une activité de 1,5 x 10⁷ U BRMP/mg pour l'IL2 réduite de départ.

## Revendications

1. Procédé de préparation d'une protéine choisie parmi les cytokines, comportant au moins un pont disulfure intramoléculaire, et éventuellement une ou plusieurs cystéines supplémentaires, comprenant l'oxydation de la protéine recombinante réduite correspondante à l'aide d'un agent d'oxydation et caractérisé en ce que l'on fait réagir une solution aqueuse de la protéine réduite à un pH inférieur à 5,0.

2. Procédé selon la revendication 1 caractérisé en ce que la protéine réduite a la séquence en acides aminés d'une protéine naturelle, d'allèles ou de dérivés de celle-ci.

3. Procédé selon l'une des revendications 1 à 2, caractérisé en ce que le pH est compris entre 0,5 et 5,0, de préférence environ 3,0.

4. Procédé selon l'une des revendications 1 à 2, caractérisé en ce que la concentration de la protéine est comprise entre 0,05 mg/ml et 50 mg/ml, de préférence entre 0,1 et 10 mg/ml.

5. Procédé selon l'une quelconque des revendications 1 à 4, caractérisé en ce que la protéine est une IL2 humaine recombinante réduite non glycosylée ayant au moins 2 cystéines en position 58 et 105.

6. Procédé selon l'une quelconque des revendications 1 à 4 caractérisé en ce que la protéine est une IL2 humaine recombinante réduite non glycosylée ayant 3 cystéines en position 58, 105 et 125 et qui manifeste une activité biologique comparable à celle de l'IL2 oxydée correspondante ayant un pont disulfure en position 58-105.

7. Procédé selon la revendication 6 caractérisé en ce que l'on fait réagir l'IL2 réduite en solution à environ 1 mg/ml, à un pH environ égal à 3,0, à une température environ égale à 37°C, en présence d'air avec du sulfate cuivrique à une concentration à environ 66 µM.

## Patentansprüche

1. Verfahren zur Herstellung eines Proteins, ausgewählt aus Cytokinen mit einer intramolekularen Disulfidbrücke und gegebenenfalls einem oder mehreren zusätzlichen Cystein(en), wobei man das entsprechende rekombinante reduzierte Protein mit einem Oxidationsmittel oxidiert, und dadurch **gekennzeichnet,** daß man eine wäßrige Lösung des reduzierten Proteins bei einem pH-Wert unter 5,0 umsetzt.

2. Verfahren nach Anspruch 1, dadurch **gekennzeichnet**, daß das reduzierte Protein die Aminosäuresequenz eines natürlichen Proteins, eines Allels oder Derivats davon besitzt.

3. Verfahren nach einem der Ansprüche 1 bis 2, dadurch **gekennzeichnet,** daß der pH-Wert zwischen 0,5 und 5,0, bevorzugt bei etwa 3,0, liegt.

4. Verfahren nach einem der Ansprüche 1 bis 2, dadurch **gekennzeichnet,** daß die Konzentration des Proteins zwischen 0,05 mg/ml und 50 mg/ml, bevorzugt zwischen 0,1 und 10 mg/ml, liegt.

5. Verfahren nach einem der Ansprüche 1 bis 4, dadurch **gekennzeichnet,** daß das Protein ein nicht glykosyliertes, reduziertes, rekombinantes menschliches Il-2 mit mindestens zwei Cysteinen in Position 58 und 105 ist.

6. Verfahren nach einem der Ansprüche 1 bis 4, dadurch **gekennzeichnet,** daß das Protein ein nicht glykosyliertes, reduziertes, rekombinantes menschliches Il-2 mit mindestens drei Cysteinen in Position 58, 105 und 125 ist und eine biologische Aktivität zeigt, die der des entsprechenden oxidierten Il-2 mit einer Disulfidbrücke in Position 58-105 vergleichbar ist.

7. Verfahren nach Anspruch 6, dadurch **gekennzeichnet,** daß man reduziertes Il-2 in Lösung mit einer Konzentration von etwa 1 mg/ml bei einem pH-Wert von etwa 3,0, bei einer Temperatur von etwa 37°C, in Gegenwart von Luft mit Kupfer(II)-sulfat in einer Konzentration von etwa 66 µM umsetzt.

## Claims

1. Preparation process for a protein chosen from the cytokines, containing at least one intramolecular disulphide bridge, and optionally one or more additional cysteines, including the oxidation of the corresponding reduced recombinant protein using an oxidizing agent and characterized in that an aqueous solution of the reduced protein is reacted at a pH of lower than 5.0.

2. Process according to claim 1, characterized in that the reduced protein has the amino acid sequence of a natural protein, of alleles or of derivatives of this.

3. Process according to one of claims 1 to 2, characterized in that the pH is comprised between 0.5 and 5.0, preferably about 3.0.

4. Process according to one of claims 1 to 2, characterized in that the concentration of the protein is comprised between 0.05 mg/ml and 50 mg/ml, preferably between 0.1 and 10 mg/ml.

5. Process according to any one of claims 1 to 4, characterized in that the protein is a non-glycolsylated reduced recombinant human IL2 having at least 2 cysteines in position 58 and 105.

6. Process according to any one of claims 1 to 4, characterized in that the protein is a non-glycolsylated reduced recombinant human IL2 having 3 cysteines in position 58, 105 and 125 and which exhibits a biological activity comparable to that of the corresponding oxidized IL2 having a disulphide bridge in position 58-105.

7. Process according to claim 6, characterized in that the reduced IL2 is reacted in solution at about 1 mg/ml at a pH approximately equal to 3.0, at a temperature approximately equal to 37°C, in the presence of air with cupric sulphate at a concentration of approximately 66 µM.
